# FASCICULE DE BREVET EUROPEEN

(11) **EP 2 493 398 B1**
(45) Date de publication et mention de la délivrance du brevet: **16.12.2015**
(21) Numéro de dépôt: 09745025.8
(22) Date de dépôt: 28.10.2009
(51) Int. Cl.: A61B 17/17

(54) **DISPOSITIF POUR LE POSITIONNEMENT ET LE REGLAGE D'UN AXE DE VISEE**
VORRICHTUNG ZUR POSITIONIERUNG UND EINSTELLUNG EINER SICHTACHSE
DEVICE FOR POSITIONING AND ADJUSTING A VIEWING AXIS

(43) Date de publication de la demande: 05.09.2012
(73) Titulaire: Chirmat Sàrl, 1870 Monthey (CH)
(72) Inventeur: ARLETTAZ, Yvan, 1870 Monthey (CH); BONJOUR, Christian, 1268 Begnins (CH)
(74) Mandataire: P&TS SA (AG, Ltd.)
(86) Numéro de dépôt international: PCT/EP2009/064234
(87) Numéro de publication internationale: WO 2011/050842

(56) Documents cités:
- EP-A1- 0 187 283
- WO-A1-2008/017501
- WO-A2-2009/109371
- US-A1- 2006 015 101

## Description

### Domaine technique

La présente invention concerne un perfectionnement aux dispositifs de positionnement d'un axe de visée, notamment pour la localisation d'un trou distal dansun clou intramédullaire pour le traitement chirurgical de fractures d'os longs.

### Etat de la technique

Les clous centromédullaires sont utilisés dans le domaine chirurgical pour stabiliser lesfracturesdesoslongs. Ce type de clou s'introduit dans les os longs, par exemple le fémur, présentant des cavités centrales dans lesquelles ils peuvent être logés en perçant simplement les extrémitéslongitudinalesde l'os.

Lorsqu'un chirurgien veut introduire un clou intramédullaire dans un oslong, par exemple le fémur, il en perce d'abord une extrémité longitudinale, en général au niveau de la fesse, et enfonce le clou dans la cavité centrale de l'os. Afin d'empêcher lesmouvementsde torsion de ce clou intramédullaire ainsi positionné, ces clous contiennent destrousde fixation à leursextrémitésproximaleset distales, dans lesquels des vis ou des clavettes doivent être introduites transversalement pour supprimer ces mouvementsde torsion indésirables pour une guérison rapide. La difficulté majeure pour les chirurgiens dans la détermination de la position et de l'axe du trou de fixation distal provient de l'incertitude liée à la déformation subie par le clou lorsde son introduction dans l'os, car le clou possède évidemment des propriétés élastiques pour pouvoir suivre la trajectoire de la cavité de l'os.

Le chirurgien orthopédiste doit d'une part localiser l'endroit du trou, et d'autre part déterminer l'axe du trou lorsdu perçage de l'os nécessaire avant d'introduire la vis ou la clavette. L'emplacement et la direction de perçage dépend de la déformation subie par le clou pour suivre la forme de la cavité de l'os. Il est donc nécessaire de retrouver la position et l'orientation du trou après l'introduction du clou dans l'os.

De nombreux systèmes existent pour aider les chirurgiens lors de cette opération et les assister lorsdu perçage. Traditionnellement, la position du clou est déterminée par exposition aux rayons X, ce qui nécessite cependant une infrastructure complexe qui n'est pas partout disponible.

Des appareils électroniques interviennent souvent pour la visualisation de coordonnéesde visée, comme par exemple dans US4621628 ou encore US5411503. Ces appareils électroniques nécessitent la présence d'une source de courant qui n'est pas toujours disponible. De tels appareils nécessitent de plus une maîtrise de la complexité inhérente à leur utilisation et donc un entraînement approprié. Ils s'avèrent par ailleurs souvent trèsonéreux.

Des solutions faisant intervenir desaimantssont apparuespour permettre un réglage nécessitant moins d'appareillage électronique pour l'affichage, le traitement du signal, et la connectique et ainsi réduire les coûtsopératoires. US5049051 et US5514145 décrivent par exemple un système mettant en oeuvre un ou plusieursaimantsà l'intérieur du clou intramédullaire et qui coopèrent avec un système de visée, afin de localiser précisément le trou avant le perçage. Ces méthodes utilisant des appareils de visée essentiellement mécaniques ne garantissent cependant pas une précision optimale, notamment pour l'alignement de l'axe de perçage par rapport au trou.

Dans la plupart des cas, la perceuse est couplée à un système de visée basé sur un aimant pivotant qui détermine un axe de perçage réglable selon 6 degrésde libertés. L'aimant permet certesde localiser précisément la position du trou, maisseslignesde champ étant omnidirectionnelles et isotropes, elles n'indiquent pas la direction de l'axe du trou. Dès lors, l'angle de perçage peut être oblique par rapport à l'axe du trou sansque ce système ne puisse ni l'indiquer ni le corriger.

Différents documents décrivent des supports qui permettent de solidariser le système de visée et de perçage par rapport au clou. US6162228 et WO9814121 décrivent de telssupportsen forme de U, avec un rail essentiellement parallèle au clou intramédullaire. Le support permet d'orienter le viseur qui peut pivoter afin de l'orienter selon l'axe du trou de fixation traversant le clou.

Ces dispositifs permettent certes d'éviter un perçage oblique, mais ne sont d'aucune aide lorsque le trou ne se trouve pasà l'endroit attendu dans le plan.

Par ailleurs, US6162228 nécessite d'orienter correctement l'aimant à l'intérieur du clou afin de positionner sespôlespar rapport à l'axe du trou de fixation. Il est difficile de positionner correctement un aimant amovible à l'intérieur d'un clou déformé.

Un tel réglage en rotation seule ne permet paspar ailleurs d'adapter de manière satisfaisante l'axe de visée lorsque le clou intramédullaire est courbé selon la forme de l'osdu patient, notamment lorsque cette déformation s'effectue dansun plan perpendiculaire à l'axe du trou de fixation.

D'autre part, US6162228 utilise une boussole électronique pour générer un signal visuel ou acoustique. Lescoûtsde fabrication d'une telle boussole s'avèrent élevés; elle s'avère par ailleurs difficile à stériliser pour un usage répété.

De nombreux dispositifsde l'art antérieur permettent un réglage de la position et de l'orientation du viseur et de la perceuse selon un nombre important de degrés de liberté. Le chirurgien orthopédiste a ainsi de nombreuses possibilités de réglage du dispositif afin de trouver la position et l'orientation appropriée. Il en résulte un temps de réglage important ce qui rallonge inutilement la durée de l'opération et complique la rééducation.

D'autres dispositifs présentent au contraire des possibilités de réglage trop limitées qui ne permettent pas un réglage précis de l'orientation ou de la position. Il en résulte fréquemment la nécessité pour le chirurgien-orthopédiste de faire un trou dans l'os qui n'est pas parfaitement aligné avec le trou à travers le clou. La vis ou la clavette est alors difficile à insérer, et le chirurgien doit faire une incision inutilement large et difficile à cicatriser pour permettre l'introduction de cette vis ou clavette.

WO 2009/109371 décrit un dispositif de visée pour aligner un trou de guidage avec un trou dans un clou intramédullaire.

EP0187283 A1 décrit un dispositif selon le préambule de la revendication 1.

### Brève description de l'invention

La présente invention a pour but la réalisation d'un dispositif pour le positionnement et l'alignement d'un axe de visée par rapport à un orifice de fixation distal d'un clou intramédullaire exempt des limitations connues de l'état de l'art.

Un but est aussi de proposer un dispositif de positionnement et d'alignement d'un axe de visée par rapport à un orifice de fixation distal, qui permet au chirurgien de déterminer précisément la position et l'orientation de l'orifice avec un minimum de réglages, selon un nombre limité de degrés de liberté.

L'invention part de la constatation que les os longs, notamment le fémur, de différents individus ont une forme qui varie peu. Les déformations du clou à l'intérieur de la cavité médullaire de l'os sont donc prévisibles, en sorte que la position et l'orientation de l'orifice de fixation distal peut être retrouvée avec précision avec un nombre de réglages limités selon un nombre limité de degrés de liberté.

L'invention part en particulier de la constatation que des réglages dans un seul plan perpendiculaire à l'axe du viseur/de la perceuse sont suffisantspour aligner l'axe de perçage avec le trou distal à travers le clou intramédullaire.

L'invention part aussi de la constatation que l'orientation de l'axe de l'orifice distal change très peu même lorsque le clou se déforme. Les changementsd'orientation dus notamment à la courbure du clou sont limités, et n'empêchent pasun perçage ni l'insertion d'une visou d'une clavette en tenant compte du jeu existant. Il n'est donc pasnécessaire de prévoir un réglage de l'inclinaison de l'axe du viseur ; une telle possibilité de réglage est même préjudiciable, car elle rend le réglage plus complexe et plus lent.

Il est donc possible de réaliser un support qui permette un réglage fin du viseur et de la perceuse selon le ou les degrés de libertés les plus pertinents, et de bloquer les translations ou rotations selon tous les autresdegrésde liberté. Dansune variante préférentielle, le viseur et la perceuse peuvent donc uniquement se déplacer par des mouvementsde translation et/ou de rotation dans un plan perpendiculaire à l'axe longitudinal du trou dansle clou non déformé ; le support empêche tous les autres déplacements par rapport au clou. On exploite donc la connaissance a priori de la position et de l'orientation théorique du trou.

Dans un premier mode de réalisation, les réglages fins se font selon un seul degré de liberté, par translation du viseur dans un plan perpendiculaire à l'axe de visée et de perçage, et selon une direction de translation perpendiculaire à l'axe du clou non déformé. Cette variante présente l'avantage d'une réalisation très simple, puisque le dispositif nécessite uniquement un moyen de réglage fin selon un seul axe, linéaire de surcroit. Cette variante est donc à la foiséconomique et facile à utiliser.

Dansun deuxième mode de réalisation, les réglages se font par rotation dansle plan décrit ci-dessuset autour d'un axe parallèle à l'axe de visée et de perçage. Cette variante est également économique et simple à utiliser ; elle présente en outre l'avantage de s'adapter à desdéformations plusimportantesdu clou.

Un degré de réglage fin par translation du viseur parallèlement à l'axe du clou peut être prévu. Cette variante renchérit le dispositif, et rend le réglage plus complexe, maisoffre le maximum de flexibilité et permet d'aligner avec précision le viseur et l'orifice du clou même en casde flexion ou déformation importante du clou.

Le dispositif permet donc d'aligner le viseur avec l'axe de l'orifice distal du clou uniquement par des déplacements selon un, voire deux, degrésde liberté dansun seul plan. Ces déplacements sont particulièrement intuitifs pour le chirurgien-orthopédiste, et leur effet sur la position du pointeur dans le viseur beaucoup plus facile à prévoir que dansle casde déplacements selon d'autresdegrésde liberté en trois dimensions. Il en résulte un tempsde réglage et une durée d'opération réduits.

Par réglage fin, on entend dans la présente demande des réglagesen continu, en fonction des indications données dans le viseur du dispositif, afin d'adapter la position et l'orientation du viseur en fonction desdéformationsdu clou médullaire.

Desajustementsa priori du dispositif selon davantage de degrés de liberté sont certes possibles afin notamment d'adapter à l'avance la longueur du dispositif à différents longueurs de clou, ou afin de minimiser la distance entre le clou et le viseur en fonction de la corpulence du patient. Ces ajustements préalables ne constituent cependant pas des réglages fins et ne sont pas effectués en fonctions des indications dans le viseur.

L'invention concernedoncdifférentesvariantesd'un dispositif pour le positionnement d'un axe de visée comportant un viseur magnétique et dans lequel les seuls réglages fins possibles qui influent sur les indications du viseur se font selon un ou au maximum deux degrés de liberté dansun plan perpendiculaire à l'axe du viseur et du trou dansle clou non déformé. Les autres réglages éventuellement possibles sont soit des réglages grossiers (par exemple des réglages qui ne sont pas effectués en continu) et généralement effectués lors de la mise en place indépendamment desindicationsdu viseur, soit des ajustements sans influence significative sur les indications dans le viseur (par exemple pour rapprocher le viseur de l'os).

### Brève description des figures

Desexemplesde mise en oeuvre de l'invention sont indiquésdans la description illustrée par les figures annexées dans lesquelles:
La figure 1 illustre en perspective un dispositif selon premier mode de réalisation de l'invention.
La figure 2 illustre en perspective un dispositif selon un deuxième mode de réalisation de l'invention.
La figure 3 est une vue de dessusd'un dispositif selon le deuxième mode de réalisation de l'invention.
La figure 4 est une vue en perspective d'une des piècesdu dispositif selon le deuxième mode de réalisation de l'invention.
La figure 5 est une vue en coupe du viseur magnétique de l'invention.
La figure 6 est une vue en coupe d'un détail du viseur magnétique de l'invention.
La figure 7 est une vue de dessusd'une variante de dispositif selon le deuxième mode de réalisation de l'invention.
La figure 8 est une vue en coupe d'un détail de la variante de dispositif selon le deuxième mode de réalisation de l'invention.

### Exemple(s) de mode de réalisation de l'invention

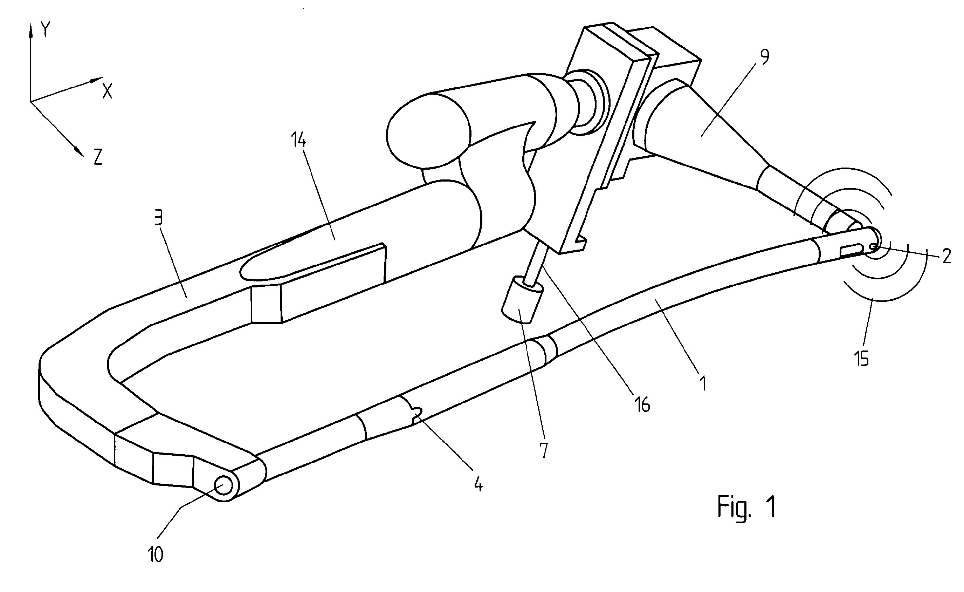

La figure 1 est une vue en perspectivesd'un dispositif de visée selon un premier mode de réalisation de l'invention. L'axe X correspond à l'axe longitudinal du clou 1 non déformé et l'axe Z (axe de visée) correspond à l'axe du viseur 9 et de l'orifice de fixation distal 2 (le trou) non déformé. Le support rigide 3 permet de positionner le viseur 9 par rapport au clou. La référence 4 indique un détrompeur 4 au niveau de la fixation du support 3 sur le clou 1.

Un manchon amovible 14 permet d'adapter la longueur du support rigide 3 en fonction de la longueur du clou employé ; il ne s'agit pasici d'un réglage fin, maisuniquement d'une possibilité d'adaptation à des clous de différentes longueurs standardisées, par pasde plusieurs centimètrestypiquement. Dansun mode de réalisation, la longueur du support rigide peut être modifiée en choisissant une pièce d'assemblage, comme le manchon 14 de la figure 1, en fonction de la longueur du clou 1.

Le viseur 9 peut être rapproché ou éloigné de l'ospar une translation en Z afin de réduire la distance entre le viseur et l'aimant dans le clou. Il ne s'agit cependant pasici d'un réglage pour centrer le viseur, mais plutôt d'une adaptation effectuée a priori pour tenir compte de la corpulence du patient. Ce réglage peut être effectué à l'avance indépendamment des indications dans le viseur 9. Même lorsde ce réglage, le viseur ne change pasd'orientation par rapport au clou. Lesdéplacements du viseur selon son axe Z longitudinal permettent donc uniquement d'améliorer l'interaction entre l'aimant du clou et l'aimant du viseur, en les rapprochant. A toutes fins utiles, ces rapprochements selon l'axe Z n'influencent cependant pratiquement pas les indications affichées par le viseur.

Le clou 1 est de préférence creux pour permettre sa déformation dans l'os et afin d'y introduire un aimant faisant office de cible pour la visée à l'intérieur, en choisissant un des trous de verrouillage distal, par exemple l'orifice de fixation distal 2. Le canal longitudinal 10 permet d'introduire une tige non représentée portant par exemple un aimant ou un élément ferromagnétique à son extrémité. On discerne les lignes de champ 15 de l'aimant qui permettront d'orienter la visée.

Dans ce mode de réalisation, le réglage fin de la position du viseur 9 se fait au moyen de la molette de réglage 7 qui permet de déplacer le viseur selon l'axe Y par rapport au clou 1. Lors du réglage, le viseur 9 se déplace donc par rapport au clou uniquement par des translations selon l'axe rectiligne Y, dans un plan perpendiculaire à l'axe Z du viseur 9 et de l'orifice 2. Les autres degrés de liberté sont bloqués. Le chirurgien orthopédiste doit ainsi uniquement actionner la molette 7 pour déplacer le viseur 9 selon l'axe Y et afin d'obtenir le meilleur alignement possible dans le viseur.

Le réglage selon l'axe Y doit être très fin car le jeu pour le positionnement de la vis ou de la clavette dans l'orifice distal de fixation 2 n'est que de l'ordre du millimètre. Il est en outre nécessaire d'empêcher un dérèglement lors du remplacement du viseur par la perceuse. Dans une variante, une tige filetée 16 solidaire de la molette 7 tourne avec elle; l'autre extrémité de cette tige est engagée dans un trou taraudé solidaire du viseur 9 (non représenté). Un ressort (non représenté) maintient une compression selon l'axe de réglage Y sur le viseur 9, de telle sorte que le viseur 9 soit engagé dans un déplacement de coulisse selon Y lorsque la tige 16 tourne avec la molette 7. La compression exercée par le ressort permet d'augmenter les forces de friction pour le maintien de molette 7 dans une position de réglage, tout en permettant néanmoins un réglage fin selon cet axe. Il est aussi possible de munir l'axe de la molette d'une vis sans fin engrenant une crémaillère solidaire du viseur.

Des tests et des études ont montré que ce réglage en Y seul est suffisant pour ajuster avec une précision remarquable l'axe du viseur 9 avec l'axe du trou 2 dans le clou 1. Cette variante offre ainsi l'avantage d'un réglage particulièrement facilité.

Dans certains cas, les déformations du clou dans le plan X-Y entraînent cependant un déplacement du trou distal selon une trajectoire circulaire avec un centre de rotation proche de l'extrémité proximale du clou 1 ou du point de rotation de la hanche. Le trou distal se déplace donc principalement selon l'axe Y, mais également, avec une moins grande amplitude, selon l'axe X. Dans une variante de ce premier mode de réalisation, l'organe de réglage permet donc de déplacer l'extrémité du viseur selon une trajectoire comportant une composante principale en Y et, simultanément, de façon dépendante, selon une composante moins importante en X, afin de suivre les déplacements de l'orifice distal. Le mouvement reste un mouvement de translation ; l'orientation du viseur n'est pas modifiée. Les deux composantes en Y et en X sont liées; on a donc un seul degré de liberté pour positionner le viseur le long d'une trajectoire courbe dans le plan X-Y.

Dans un mode de réalisation préférentiel, afin d'obtenir ces mouvements simultanés et dépendants en X et en Y, la molette 7 agit par exemple sur un coulisseau se déplaçant dans un guide ou une coulisse définissant la trajectoire désirée. Le réglage se fait à l'aide de la molette 7 en agissant selon un seul degré de liberté pour déplacer le viseur en Y, éventuellement avec une composante dépendante en X. Le réglage est donc très rapide. La compensation en X est d'autant plus efficace si l'on connaît le rayon de courbure de la déformation du clou; on pourra donc concevoir des coulisses interchangeables, par exemple des coulisses jetables, permettant d'adapter la compensation en X à la longueur du clou.

Le réglage longitudinal selon l'axe Y est efficace même si l'aimant pivote dans l'os; il est uniquement nécessaire que la position longitudinale de l'aimant corresponde à la position du trou distal. L'aimant peut ainsi parfaitement être monté à l'extrémité d'une tige souple avec un axe cylindrique, libre de tourner sur elle-même dans le trou ; les lignes de champ 15 de l'aimant, même isotropes, suffiront pour le positionnement du viseur 9 selon l'axe Y.

Lorsque le positionnement de l'axe de visée est effectué, il est possible d'entamer une étape de perçage. Dans ce but, on retire tout d'abord la tige et l'aimant du canal longitudinal dans le clou, puis on remplace le viseur 9 sur le support rigide 3 par une perceuse (non représentée) ou un autre outil.

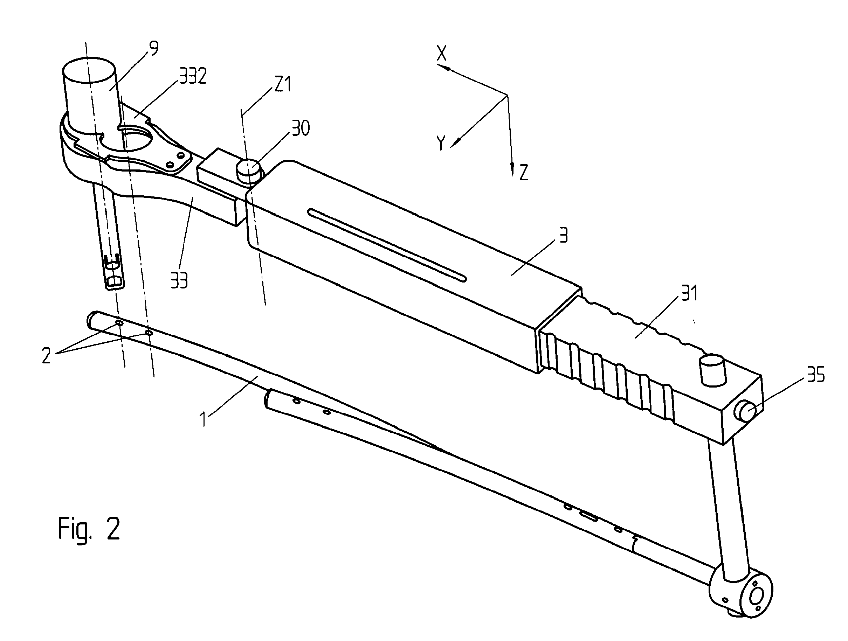

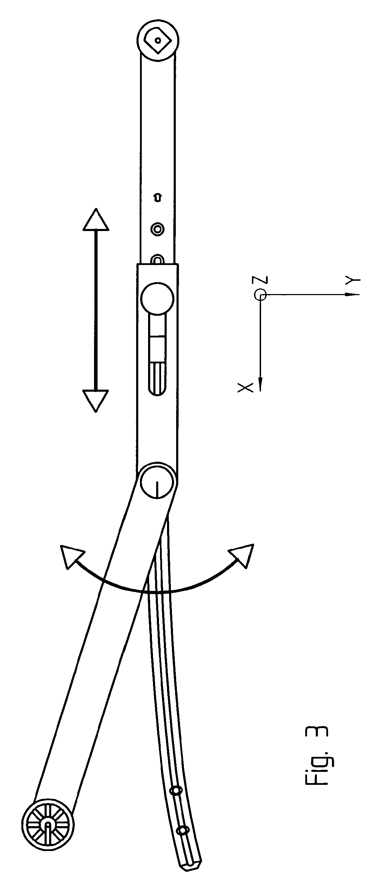

La figure 2 illustre en perspective un autre mode de réalisation de l'invention dans lequel le réglage de la position du viseur 9 se fait essentiellement par un mouvement de rotation autour d'un axe z1 parallèle à l'axe de visée et de perçage Z. Le dispositif comporte un support rigide 3, par exemple en fibre de carbone, télescopique avec une portion d'extension 31 qui permet d'adapter grossièrement la longueur du support selon l'axe X en fonction de la longueur du clou 1 ; le réglage se fait avantageusement par crans successifs, par exemple par crans espacés de 20 mm ou adaptés à la gamme de longueurs de clous disponible. Une échelle sur le tube interne 31 peut s'afficher dans une fenêtre non représentée dans le tube externe 3 afin d'indiquer la longueur actuellement choisie. Le maintien se fait avantageusement par déformation élastique (clipsage) des crans de la portion télescopique. Dans une autre variante illustrée sur la figure 3, l'indexation de la longueur d'extension des tubes télescopiques se fait au moyen d'une goupille insérée dans une paire de trous traversant les deux tubes.

Une vis de réglage 35 permet d'ajuster la distance du clou 1 en Z par rapport au support 3, en fonction de la corpulence du patient. Comme le réglage par crans, cet ajustement grossier est effectué a priori, indépendamment des indications dans le viseur 9.

L'extrémité distale 33 du support 3 est articulée par rapport au corps 3 de ce support au moyen d'un pivot 30 ; elle peut aussi pivoter autour de l'axe Z1 parallèle à l'axe de visée et de perçage Z. Dans ce mode de réalisation, le réglage se fait donc dans le plan perpendiculaire à l'axe Z du trou 2, mais par une rotation plutôt que par une translation. Cette possibilité de réglage est illustrée sur la vue de dessus de la figure 3.

Comme dans la variante de la figure 1, le viseur est démonté et remplacé par une perceuse ou un autre outil lorsque la position de perçage optimale a été trouvée.

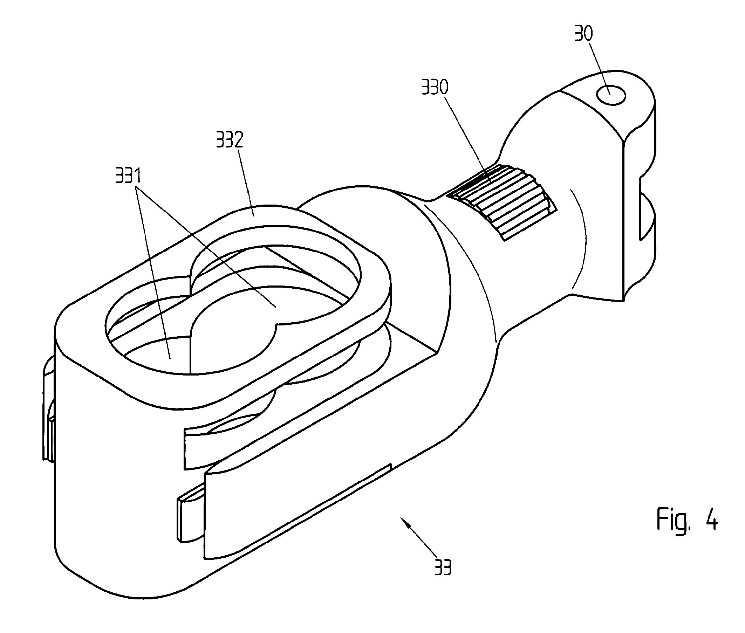

La figure 4 illustre en perspective un mode de réalisation avantageux de l'extrémité distale 33 du support rigide 3. Dans cette variante, la longueur du support 3 peut être ajustée de manière fine en X au moyen de la vis micrométrique qui permet de rallonger ou de raccourcir l'élément, afin d'effectuer une correction fine et un ajustement de la position du viseur selon l'axe X. La référence 331 indique l'orifice dans lequel le viseur, puis la perceuse, sont introduits et maintenus. L'orifice comporte avantageusement deux emplacements prédéfinis permettant de monter le viseur ou la perceuse à deux endroits différents, par exemple pour percer en regard des deux trous de l'extrémité distale du clou. La référence 332 correspond à un levier permettant de maintenir le viseur 9 et ultérieurement la perceuse dans l'orifice 331.

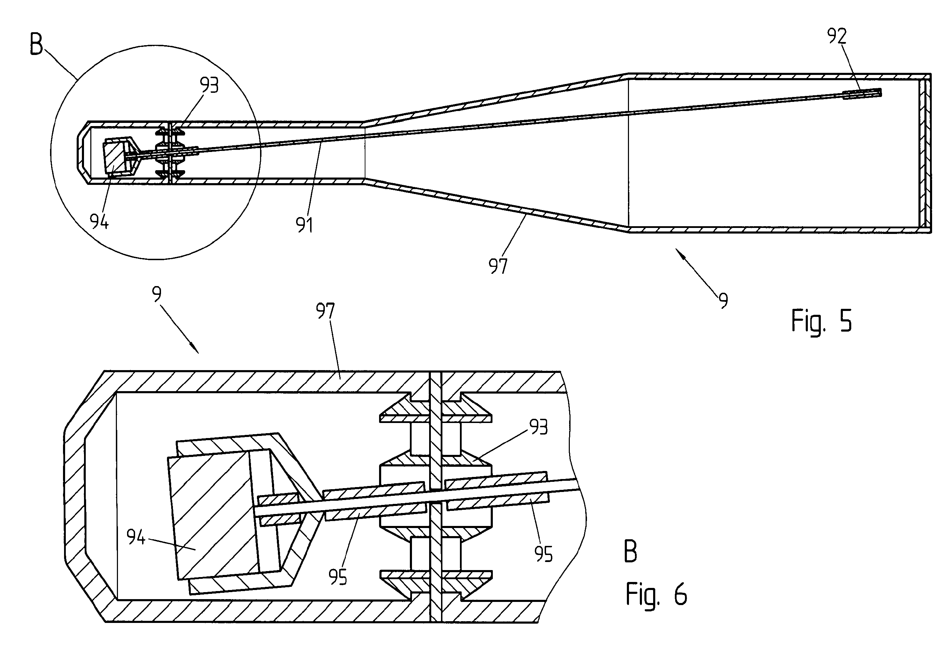

Les figures 5 et 6 illustrent une vue en coupe d'un exemple de viseur 9 selon l'invention. Il comporte une coque conique 97 avantageusement réalisée en matériau synthétique, par exemple en homo-polymère, moulé ou injecté. L'axe de la coque détermine l'axe de visée (Z). Une des extrémités de la coque est fermée par une vitre ou une surface de visée en matériau plastique transparent clipsée dans la coque ; cette surface de visée permet au chirurgien d'observer au travers la position d'un pointeur 92 fixé à l'extrémité d'une tige 91 et indiquant le réglage à effectuer pour amener le viseur en regard de l'aimant dans le clou. La tige 91 est avantageusement réalisée en carbone ou dans un autre matériau amagnétique. Le pointeur 92 peut être réalisé par exemple au moyen d'une gaine thermorétractable fixée sur la tige, par exemple une gaine de couleur vive.

La tige 91 est articulée sur un palier 93 clipsé dans le viseur 9. L'autre extrémité de la tige est munie d'un aimant 94 dont la position est influencée par le champ magnétique 15 émis par l'aimant dans le clou 2. Les références 95 désignent des douilles enfilées sur la tige 91 de part et d'autre du palier, et qui constituent des butées pour empêcher le coulissement de cette tige par rapport au palier, sans empêcher son pivotement. Le point de pivot peut par exemple consister en un simple trou dans le palier 93. L'ensemble des pièces formant le viseur sont de préférence fixées à la coque 97 par clipsage ou déformation élastique.

Lorsque l'aimant pivotant 94 s'oriente vers l'aimant dans le clou, le pointeur 92 à l'extrémité de la tige 91 opposée à l'os se déplace dans l'objectif du viseur 9 de façon correspondante. L'objectif comporte par exemple des cercles concentriques sur une surface transparente, et dessine ainsi une cible dans laquelle se déplace le pointeur 92 pour indiquer la position de l'orifice distal de fixation 2. Le réglage de la molette 7, respectivement du pivot 30 et de la molette 330, entraîne un déplacement du viseur 9 dans le plan X-Y. L'aimant 94 pivote pour s'orienter vers l'aimant du clou, entraînant le pointeur 92.

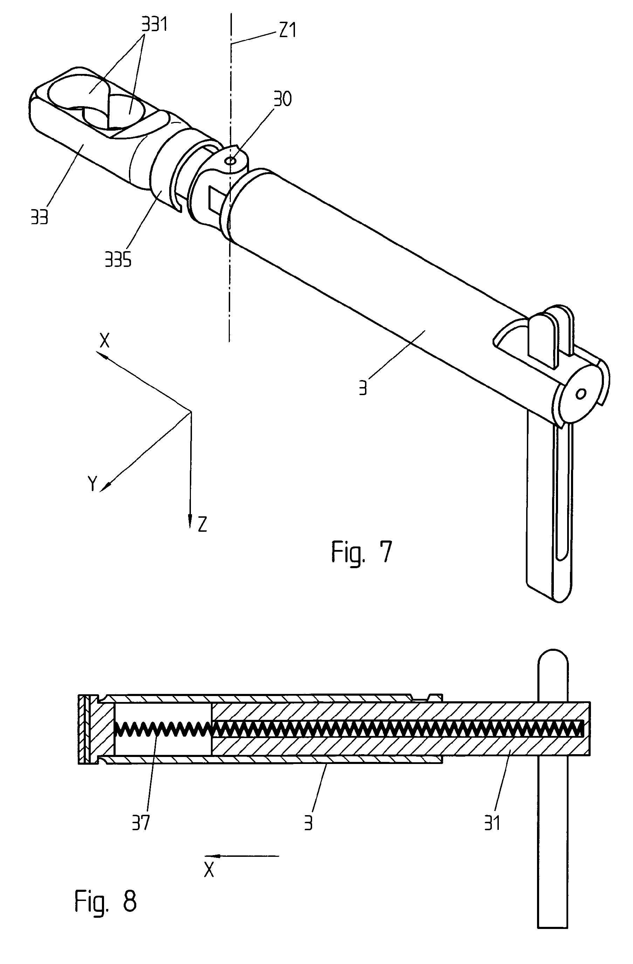

La figure 7 illustre en perspective une seconde variante de dispositif selon le deuxième mode de réalisation. Les mouvements possibles du viseur non représenté à insérer dans le logement 331 de l'extrémité distale 33 sont les mêmes que dans la première variante décrite ci-dessus en relation avec les figures 2 à 6 ; le viseur peut également pivoter autour de l'axe Z1 parallèle à l'axe de visée et de perçage Z, au moyen d'un pivot 30. Une bague 335 peut être dessérée pour modifier la longueur de l'extrémité distale 33, puis resserrée pour bloquer la position choisie.

Cette variante se distingue avant tout de la première variante ci-dessus par le corps 3 du support rigide, qui est formé ici d'un tube télescopique de section cylindrique. Un ressort longitudinal 37 (figure 8) est logé et constamment en compression à l'intérieur du tube interne 31, afin de rigidifier l'arrangement télescopique et de garantir une précision de positionnement à chaque position d'indexation des tubes. L'adaptation de la longueur du tube 3 à la longueur du clou se fait avantageusement au moyen d'une goupille liée au tube externe et qui suit une fente à travers le tube interne comportant plusieurs positions d'arrêt tous les 20 millimètres par exemple.

Le dispositif peut aussi être adapté à la pose de tous clous centromédullaire ayant un canal longitudinal de guidage, ainsi qu'à la pose de clous dépourvus d'un tel canal, y compris à la pose de prothèses longues. En particulier, le dispositif peut être adapté à la pose de clous plats sans cavités intégrant un aimant dans la masse du clou, ou de clous extendeurs. Dans ce cas, le trou à travers le clou n'est généralement pas percé, mais son emplacement est prévu à l'avance ; le perçage du clou est effectué avec le perçage de l'os, à cet emplacement et dans une direction perpendiculaire à la surface du clou. Le dispositif permet dans ce cas le positionnement de l'axe de visée par rapport à l'orifice de fixation distal prévu dans le clou intramédullaire. Si l'aimant est monté dans la masse du clou à distance de l'endroit prévu pour le perçage, il sera nécessaire de monter la perceuse sur le support à distance de l'endroit repéré avec le viseur. L'aimant peut aussi être clipsé dans un trou du clou, et retiré avant ou lors du perçage.

Ce dispositif s'avère être particulièrement adapté pour diminuer les coûts opératoires et simplifier le mode opératoire grâce à l'utilisation de pièces peu coûteuses, faciles à usiner et modulaires. La modularité des pièces, par exemple pour les séquences de visée et de perçage, permet de nettoyer et stériliser plus facilement les différentes pièces lorsqu'elles sont prises séparément que pour un appareil où il est impossible de désolidariser les pièces constitutives du dispositif, notamment celles destinées à la visée et celles destinées au perçage. La plupart des pièces qui sont réutilisées, notamment le support rigide 3 et éventuellement la tige avec l'aimant, peuvent ainsi être conçues avec une section cylindrique permettant une stérilisation efficace. Les autres pièces, notamment le viseur 9 et le manchon 14, sont de préférence jetables.

L'utilisation d'un viseur 9 et/ou d'un manchon 14 jetables, constituésuniquement de pièces mécaniques, est indépendante des déplacements du viseur 9 restreintsselon l'axe Z.

## Revendications

1. Dispositif pour le positionnement d'un axe de visée par rapport à un orifice de fixation distal (2) d'un clou intramédullaire (1), comprenant un viseur (9) et un support rigide (3),
ledit viseur comportant un axe (Z) longitudinal,
ledit support rigide (3) assurant le positionnement dudit viseur (9) par rapport audit clou intramédullaire (1), dans lequel la position du viseur peut être réglée en fonction des indications dans ledit viseur par des translations et/ou rotations du viseur dans un plan perpendiculaire à son dit axe (Z) longitudinal, lesdites rotations étant effectuées selon un axe (Z1) lié au support rigide et parallèle audit axe (Z) longitudinal du viseur (9), par un pivot (30) permettant à l'extrémité distale (33) dudit support rigide (3) de pivoter par rapport au reste dudit support rigide (3), **caractérisé par** une vis micrométrique pour régler en continu la longueur dudit support fixe (3).

2. Le dispositif de la revendication 1, **caractérisé en ce que** la position du viseur peut être réglée de manière fine en fonction des indications dans ledit viseur uniquement par des translations selon un seul axe (Y) dans ledit plan perpendiculaire à son dit axe (Z) longitudinal.

3. Le dispositif de la revendication 2, **caractérisé en ce que** la position du viseur peut être réglée en fonction des indications dans ledit viseur uniquement par des translations selon un axe (Y) perpendiculaire audit clou (1).

4. Le dispositif de la revendication 1, **caractérisé en ce que** la position du viseur (9) est réglable de manière fine en fonction des indications dans ledit viseur uniquement par des rotations dans ledit plan perpendiculaire à son dit axe (Z) longitudinal.

5. Le dispositif de la revendication 1, comportant une molette (330) agissant sur la vis micrométrique pour régler en continu la longueur dudit support fixe (3).

6. Le dispositif de l'une des revendications 1 à 5, permettant des translations dudit viseur (9) selon son dit axe (Z) longitudinal afin de le rapprocher du clou en tenant compte de la corpulence du patient et dans lequel lesdits déplacements du viseur selon son axe (Z) longitudinal n'influencent pas sur les indications affichées par le viseur (9).

7. Dispositif selon l'une des revendications 1 à 6, le viseur (9) comprenant un aimant pivotant (94) par rapport au support (3) et coopérant avec un aimant logé dans ledit clou (1), ledit aimant pivotant (94) étant lié à une tige (91) pivotant autour d'un point fixe (93) du viseur, l'extrémité de la tige opposée audit aimant pivotant étant munie d'un pointeur (92).

8. Dispositif selon la revendication 7, ledit viseur comportant :
une coque (97) en matériau synthétique ;
un palier (93) inséré dans ladite coque (97) et maintenant ladite tige;
une surface de visée insérée dans ladite coque.

9. Dispositif selon l'une des revendications 1 à 8, ledit support rigide (3) comportant un détrompeur (4) coopérant avec ledit clou (1) pour déterminer un axe de visée parallèle à celui dudit orifice de fixation distal.

10. Dispositif selon l'une des revendications 1 à 9, la longueur dudit support rigide (3) pouvant être ajustée par crans par adjonction d'une pièce additionnelle d'assemblage amovible (14) pour collaborer avec des clous de longueurs différentes.

## Patentansprüche

1. Vorrichtung zur Positionierung einer Sichtungsachse in Bezug auf ein distales Fixierungsloch (2) für einen Marknagel (1), mit einer Sichtvorrichtung (9) und einer stabilen Stütze (3),
wobei die benannte Sichtvorrichtung eine Längsachse (Z) enthält;
wobei die benannte stabile Stütze (3) die Positionierung der benannten Sichtvorrichtung (9) in Bezug auf den Marknagel (1) sicherstellt,
worin die Position der Sichtvorrichtung angepasst werden kann, abhängig von Angaben in der benannten Sichtvorrichtung durch Translations- und/oder Rotationsbewegungen der Sichtvorrichtung in einer zu ihrer benannten Längsachse (Z) senkrechten Ebene, wobei die benannten Rotationen entlang einer Achse (Z1) ausgeführt werden, die verbunden ist mit der stabilen Stütze und parallel zur benannten Längsachse (Z) der Sichtvorrichtung (9), mittels eines Drehgelenks (30), welches dem distalen Ende (33) der benannten stabilen Stütze (3) erlaubt, in Bezug auf den Rest der benannten stabilen Stütze zu schwenken, **gekennzeichnet durch** eine Mikrometerschraube zur kontinuierlichen Anpassung der Länge der benannten stabilen Stütze (3).

2. Die Vorrichtung von Anspruch 1, **dadurch gekennzeichnet, dass** die Position der Sichtvorrichtung abhängig von Angaben in der benannten Sichtvorrichtung nur durch Translationen entlang einer einzigen Achse (Y) in der benannten Ebene senkrecht zu ihrer benannten Längsachse (Z) genau angepasst werden kann.

3. Die Vorrichtung von Anspruch 2, **dadurch gekennzeichnet, dass** die Position der Sichtvorrichtung abhängig von Hinweisen in benannter Sichtvorrichtung nur durch die Translationen entlang einer Achse (Y) senkrecht zu benanntem Nagel (1) angepasst werden kann.

4. Die Vorrichtung von Anspruch 1, **dadurch gekennzeichnet, dass** die Position der Sichtvorrichtung (9) abhängig von Angaben in der benannten Sichtvorrichtung nur durch Rotationen in der benannten Ebene senkrecht zu ihrer benannten Längsachse (Z) genau angepasst werden kann.

5. Die Vorrichtung von Anspruch 1, mit einem Daumenrad (330) zur Betätigung der Mikrometerschraube zur kontinuierlichen Anpassung der Länge der benannten stabilen Stütze (3).

6. Die Vorrichtung einer der Ansprüche 1 bis 5, welche Translationen der benannten Sichtvorrichtung (9) entlang ihrer benannten Längsachse (Z) erlaubt, um sie dem Nagel näher zu bewegen, unter Berücksichtigung der Statur des Patienten, und worin die benannten Verschiebungen der Sichtvorrichtung entlang ihrer benannten Längsachse (Z) die Angaben, welche durch die Sichtvorrichtung (9) angezeigt werden, nicht beeinflussen.

7. Vorrichtung gemäss einem der Ansprüche 1 bis 6, wobei die Sichtvorrichtung (9) einen Magnet (94) enthält, der relativ zur Stütze (3) dreht und mit einem in benanntem Nagel (1) eingelegten Magnet zusammenarbeitet, wobei der benannte drehende Magnet (94) mit einem um einen fixierten Punkt der Sichtvorrichtung drehenden Stab (91) verbunden ist, wobei die Stabextremität gegenüber dem benannten drehenden Magnet mit einem Zeiger (92) ausgestattet ist.

8. Vorrichtung gemäss Anspruch 7, wobei die benannte Sichtvorrichtung enthält:
eine Schale (97) aus Kunststoff;
eine Lagerung (93), welche in die benannte Schale (97) eingeführt wird und den benannten Stab hält;
eine in die benannte Schale eingeführte Sichtoberfläche (90).

9. Vorrichtung gemäss einem der Ansprüche 1 bis 8, wobei die benannte stabile Stütze (3) eine Unverwechselbarkeitseinrichtung (4) aufweist, die mit dem benannten Nagel (1) zusammenarbeitet, um eine Sichtachse zu bestimmen, welche parallel zur derjenigen des benannten distalen Fixierungslochs ist.

10. Vorrichtung gemäss einem der Ansprüche 1 bis 9, wobei die Länge der benannten stabilen Stütze (3) schrittweise mittels Anfügung eines zusätzlichen abnehmbaren Bauteils (14) angepasst werden kann, um mit Nägeln von unterschiedlichen Längen zusammenzuarbeiten.

## Claims

1. Device for positioning a viewing axis in relation to a distal fixation hole (2) for an intramedullary nail (1), comprising a viewing device (9) and a rigid support (3),
wherein said viewing device comprises a longitudinal axis (Z);
wherein said rigid support (3) ensures the positioning of said viewing device (9) relative to the intramedullary nail (1),
wherein the position of the viewing device can be adjusted depending on indications in said viewing device by translational and/or rotational movements of the viewing device in a plane perpendicular to its said longitudinal axis (Z), wherein said rotational movements are performed along an axis (Z1) connected to the rigid support and parallel to said longitudinal axis (Z) of the viewing device (9), by a pivot (30) enabling the distal extremity (33) of said rigid support (3) to pivot relative to the remainder of said rigid support (3), **characterized by** a micrometric screw for adjusting in a continuous fashion the length of said fixed support (3).

2. The device of claim 1 **characterized in that** the position of the viewing device can be precisely adjusted depending on indications in said viewing device only by translational movements along a single axis (Y) in said plane perpendicular to its said longitudinal axis (Z).

3. The device of claim 2, **characterized in that** the position of the viewing device can be adjusted depending on the indications in said viewing device only by translational movements along an axis (Y) perpendicular to said nail (1).

4. The device of claim 1, **characterized in that** the position of the viewing device (9) can be precisely adjusted depending on indications in said viewing device only by rotational movements in said plane perpendicular to its said longitudinal axis (Z).

5. The device of claim 1, comprising a thumb wheel (330) actuating the micrometric screw for adjusting in a continuous fashion the length of said fixed support (3).

6. The device of one of the claims 1 to 5, enabling translational movements of said viewing device (9) along its said longitudinal axis (Z) in order to move it closer to the nail, taking into account the patient's build, and wherein said displacements of the viewing device along its longitudinal axis (Z) do not influence the indications displayed by the viewing device (9).

7. Device according to one of the claims 1 to 6, wherein the viewing device (9) comprises a magnet (94) pivoting relative to the support (3) and working together with a magnet lodged in said nail (1), wherein said pivoting magnet (94) is connected to a rod (91) pivoting around a fixed point (93) of the viewing device, wherein the extremity of the rod opposite said pivoting magnet is provided with a pointer (92).

8. Device according to claim 7, said viewing device comprising:
a shell (97) of synthetic material;
a bearing (93) inserted into said shell (97) and holding said rod;
a viewing surface (90) inserted into said shell.

9. Device according to one of the claims 1 to 8, wherein said rigid support (3) comprises a shape-keyed connection (4) working with said nail (1) to determine a viewing axis parallel to the one of said distal fixation hole.

10. Device according to one of the claims 1 to 9, wherein the length of said rigid support (3) can be adjusted by increments by adding an additional removable assembly part (14) to work with nails of different lengths.
